# EUROPÄISCHE PATENTSCHRIFT

(11) **EP 3 479 861 B1**
(45) Veröffentlichungstag und Bekanntmachung des Hinweises auf die Patenterteilung: **03.08.2022**
(21) Anmeldenummer: 18000862.5
(22) Anmeldetag: 02.11.2018
(51) Int. Cl.: A61M 16/00, A61M 16/04

(54) **VORRICHTUNG ZUR BEATMUNG UNTER BERÜCKSICHTIGUNG DER EINFLÜSSE EINES TUBUS**
DEVICE FOR VENTILATION TAKING INTO ACCOUNT THE EFFECTS OF A TUBE
DISPOSITIF DE VENTILATION PRENANT EN COMPTE L'INFLUENCE D'UN TUYAU

(30) Priorität: 07.11.2017 DE 102017010269
(43) Veröffentlichungstag der Anmeldung: 08.05.2019
(73) Patentinhaber: Löwenstein Medical Technology S.A., 2557 Luxembourg (LU)
(72) Erfinder: KREMEIER, Peter, 76149 Karlsruhe (DE); GESKE, Ralf, 17039 Brunn (DE)
(74) Vertreter: Marx, Thomas

(56) Entgegenhaltungen:
- EP-A1- 2 644 220
- EP-A1- 3 061 481
- DE-A1- 4 038 871
- DE-A1- 4 116 608
- DE-A1- 19 528 113
- DE-A1-102007 047 105
- US-A1- 2010 288 283
- US-B1- 6 920 875
- GUTTMANN J ET AL: "Respiratory comfort of automatic tube compensation and inspiratory pressure support in conscious humans", INTENSIVE CARE MEDICINE, SPRINGER, BERLIN, DE, Bd. 23, Nr. 11, 1. November 1997 (1997-11-01), Seiten 1119-1124, XP019699554, ISSN: 1432-1238

## Beschreibung

Die Erfindung betrifft eine Vorrichtung zur Beatmung, umfassend eine Atemgasquelle, einen Sensor, eine Steuereinheit, wobei die Steuereinheit an mindestens einen Sensor zur Erfassung eines Messwertes angeschlossen ist, eine Einrichtung zur Verbindung mit einem Tubus, und einen Analysator. Ein Endotrachealtubus (ETT) bzw. eine Endotrachealkanüle sind eine flexible Kunststoffröhre, die für die Beatmung in der Trachea platziert wird.

Der ETT bzw. die Trachealkanüle beeinflussen, aufgrund ihrer geringen Innendurchmesser, den Gesamtwiderstand des respiratorischen Systems unter der Beatmung. Wird ein engerer Tubus verwendet, so ergibt sich eine größere Druckdifferenz bei gleichem Gasfluß. Tubusmodelle verschiedener Hersteller unterscheiden sich zudem in ihrem tubusspezifischen Widerstand. Verschiedene Verfahren wurden vorgeschlagen, um den erhöhten Widerstand des ETT zu kompensieren.

Die Tubuskompensation (ATC) der Fa. Dräger bietet eine automatische Anhebung des Drucks in Abhängigkeit von Kenndaten des ETT (Tubuslänge und/oder -durchmesser). Dazu müssen jedoch die Werte des Tubus zu Beginn eingegeben werden. Außerdem ist eine ständige Messung des Gasflusses und anschließende Nachregelung des Drucks erforderlich, was zu Regelschwingungen führen kann und die Beatmung ineffizient macht.

Dabei bleibt unberücksichtigt, dass sich unter der Beatmung Ablagerungen im ETT bilden können, die den Widerstand des ETT deutlich verändern können. Eine Längenkürzung des ETT, die der Arzt zu Beginn der Beatmung oft vornimmt, reduziert den tubusspezifischen Widerstand zudem.

Die DE 4116608 A1 schlägt zur Tubuskompensation vor, den Druck in der Lunge zu bestimmen. Dazu wird ein Drucksensor durch den Tubus bis in die Trachea vorgeschoben. Der Drucksensor erhöht den tubusspezifischen Widerstand zusätzlich. Zudem kann der Drucksensor leicht durch Schleim verstopfen.

Aus EP 3 061 481 A1 ist ein Verfahren und eine Vorrichtung zur volumenabhängigen Triggerung der Ausatemphase bekannt. US 6,920,875 B1 betrifft einen Ventilator und ein Verfahren zur Steuerung eines Ventilators bekannt, um einen Patienten mit einem gewünschten inspiratorischen Zielvolumen eines Fluids, wie beispielsweise Luft oder einer Sauerstoffmischung, zu versorgen. Es besteht daher ein Bedarf an einer einfachen Vorrichtung zur Tubuskompensation, die ohne vorherige Kenntnis der tubusspezifischen Widerstände und ohne vorherige Eingabe von Tubuskenndaten funktioniert.

Die Aufgabe der vorliegenden Erfindung ist es daher eine einfache und robuste Vorrichtung zur Tubuskompensation anzugeben. Dabei soll eine verbesserte Beatmung unter Berücksichtigung der Einflüsse eines Endotrachealtubus (ETT) oder einer Endotrachealkanüle (beide auch vereinfachend als Tubus bezeichnet) erzielt werden.

Die Aufgabe wird gelöst durch eine. Vorrichtung zur Beatmung nach Anspruch 1. Die Unteransprüche definieren bevorzugte Ausführungsbeispiele der Erfindung.

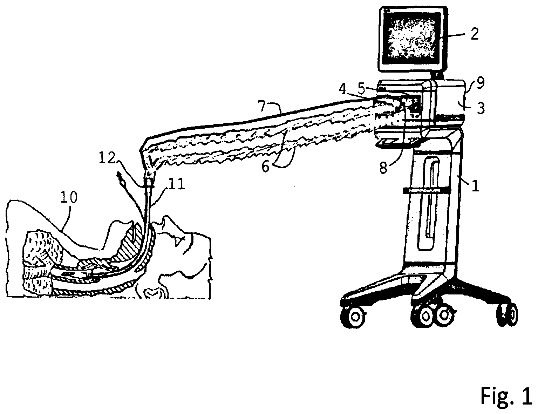

Fig. 1 zeigt den grundsätzlichen Aufbau einer erfindungsgemäßen Vorrichtung zur Beatmung. Im Bereich eines Gerätegehäuses (1) mit Bedienfeld sowie Anzeige (2) ist in einem Geräteinnenraum eine Atemgasquelle (3) angeordnet. Die Atemgasquelle kann ein Gebläse sein oder eine Druckgasquelle. Über die Kopplungen -(4,5) wird ein Schlauchsystem (6) angeschlossen. Entlang des Schlauchsystems (6) kann ein zusätzlicher Druckmessschlauch (7) verlaufen, der über einen Druckeingangsstutzen (8) mit dem Gerätegehäuse (1) verbindbar ist. Zur Ermöglichung einer Datenübertragung weist das Gerätegehäuse (1) eine Schnittstelle. (9) auf (im Bild nicht zu sehen).

Fig. 1 zeigt darüber hinaus einen intubierten Patienten (10) mit einem Endotrachealtubus (11) und einem Arischlusskonnektor (12) für das Beatmungsschlauchsystem (6).

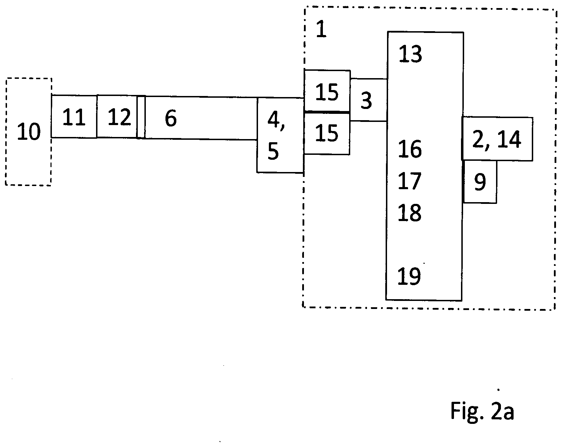

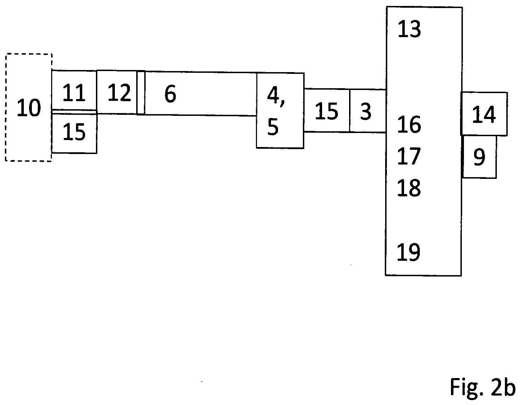

Fig. 2a und b zeigen den prinzipiellen Aufbau der steuerungstechnischen Vorrichtungskomponenten einer erfindungsgemäßen Vorrichtung.

Eine Steuereinheit (13) ist mit einem Eingabemodul (14) (nicht dargestellt) zur Dateneingabe versehen. Das Eingabemodul kann ein Touchscreen (2) sein. Über das Eingabemodul (14) können beispielsweise von einem Arzt Sollwerte für die Beatmung eingegeben werden. Die Steuereinheit (13) ist mit einem Sensor (15) verbunden, der mindestens einen Beatmungsparameter eines Patienten erfasst. Der Sensor (15) ist als ein Fluss-Sensor ausgebildet, dessen Messsignal zur Ermittlung eines Volumenwertes einem Integrator (17) zugeführt wird. Zusätzlich kann ein Drucksensor vorgesehen werden. Der oder die Sensoren können im Bereich der Vorrichtung (1) und/oder in der Nähe des Patienten (10) oder Tubus (11) vorgesehen sein. Der Analysator (18) weist eine Integratorfunktion, die die Funktion eines Integrators (17) zur Auswertung des Volumenverlaufes mit übernimmt, auf.

Dem Analysator wird auch der im Bereich des Speichers (16) abgelegte Vergleichswert für das Volumen zugeführt. Darüber hinaus ist der Analysator (18) mit einem Druckgenerator (19) gekoppelt, der einen jeweiligen Solldruck für die Atemgasquelle (3) vorgibt:

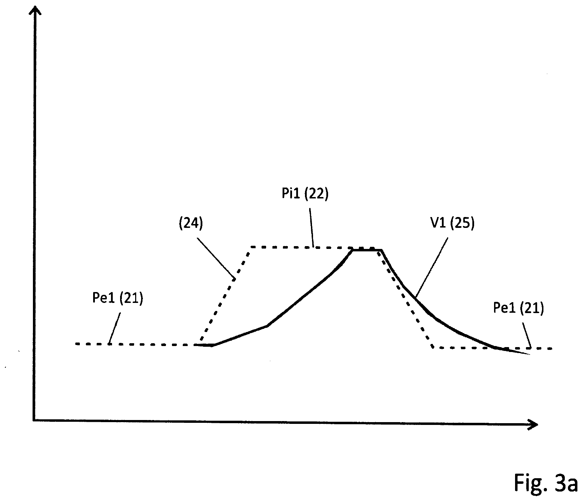

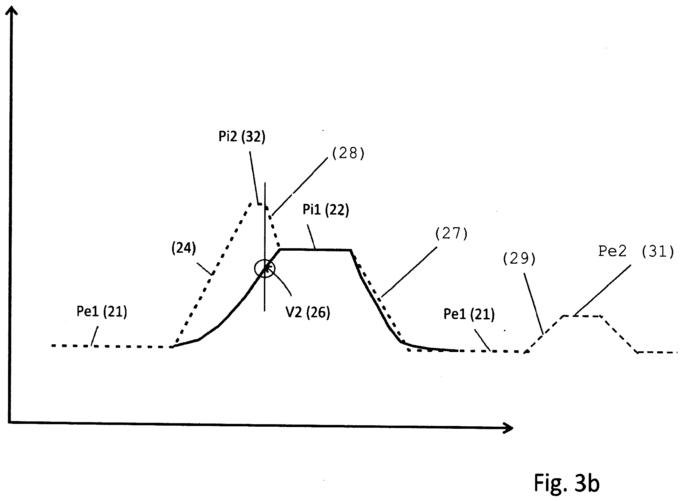

Fig. 3 zeigt den Verlauf von Druck und Volumen bei Anwendung der Tubuskompensation in einer erfindungsgemäßen Vorrichtung. Die Tubuskompensation kann beispielsweise im Rahmen eines BiLevel-Modus, oder auch bei einem patienten-getriggerten BiLevel-Modus, durchgeführt werden. Hierbei ist die Druckdifferenz vorgegeben und das Volumen ergibt sich aus der Druckdifferenz und der Gesamtcompliance.
a) Zunächst erfolgt keine Kompensation, sondern die vom Bediener eingestellte druckbasierte Beatmung mit Volumenkontrolle. Nach beispielsweise 2 bis 5 Atemhüben ist das applizierte Zugvolumen V1(25) (Volumenkontrolle) bekannt. Dieses wird als Ausgangswert verwendet. Der Druckgenerator (19) gibt den Druck initial auf ein exspiratorisches Niveau Pe1 (21) und dann auf ein inspiratorisches Niveau Pi1 (22) vor. Das Volumen ergibt sich passiv aus der vorgegebenen Druckdifferenz Pe1 - Pi1, wenn keine Volumen-basierte Beatmung eingestellt wurde. Anschließend senkt der Druckgenerator (19) den Druck auf das exspiratorische Niveau Pe1 (21) ab. Der Sensor (15) bestimmt den Gas-Fluss und der Analysator (18) das Volumen V1 (25), welches im Speicher (16) abgelegt wird.
b) Der Druckgenerator (19) gibt in einem nachfolgenden Zyklus den Druck auf dem exspiratorischen Niveau Pe1 (21) vor und hebt den Druck dann mit einem vorgebbaren Druckverlauf (24) auf ein inspiratorisches Niveau Pi2 (32) an. Der Sensor (15) bestimmt den Fluss und der Analysator (18) das Volumen V2 (26). Der Druckgenerator (19) senkt den Druck erst dann auf das Niveau Pi1 (22) ab, wenn das Volumen V2 (26) einen vordefinierten Anteil, beispielsweise 80%, des Volumens V1 (25) erreicht hat. Dazu vergleicht die Steuereinheit (13) das Volumen V1 (25) aus dem Speicher mit dem Volumen V2 (26).

Erfindungsgemäß erfolgt zunächst keine Kompensation (des Tubus), sondern die vom Bediener eingestellte druckbasierte Beatmung (mit Volumenkontrolle). Nach beispielsweise 2 bis 5 Atemhüben ist das applizierte Zugvolumen V1 (25) bekannt. Dieses wird als Ausgangswert verwendet.

Der Druck wird dann zu Beginn der nächsten Inspiration (kontrolliert) kurzfristig auf eine Höhe von Pmax - 3 mbar (Pi2 (32) angehoben. Die Druckrampe (Steilheit der Öffnung der Proportionalventile) kann dabei beispielsweise 200ms betragen oder einer Sinusfunktion oder einer beliebigen anderen Funktion, beispielsweise einer Exponentialfunktion entsprechen.

Bei diesem Druck (32) wird das applizierte Zugvolumen gemessen. Wenn dieses beispielsweise 80% des notwendigen Zugvolumens V1 (25) erreicht hat, wird der Druck auf den eingestellten Plateaudruck Pi1 (22) abgesenkt. Die weitere Inspiration erfolgt dann unter diesem Druck mit vermindertem Fluss.

Die Applikation von 80% des V1 sollte in einer vorgegebenen Zeit erfolgen. Angesetzt wird dabei eine Zeit von beispielsweise 100 - 600 ms, bevorzugt 300 ms. Stellt sich heraus, dass diese Zeit unterschritten wird, ist der Anfangsdruck Pmax-3mbar in Stufen von 1 mbar zu reduzieren, bis sich eine stabile Situation eingestellt hat.

Bei der eingestellten Druckdifferenz (BiLevel) wird der Druck Pi2 (32) zu Beginn des Atemhubes auf ein vorgegebenes Niveau von Pmax, beispielsweise Pmax-3mbar, mit einer Rampe von beispielsweise 90- 300 ms angehoben. Der maximale Druck Pmax ist dabei voreingestellt oder vom Anwender vorgebbar.

Dabei stellt sich ein, im Vergleich zu Pi1 (22), erhöhter Plateaudruck ein, bei dem das Volumen noch nicht vollständig appliziert ist. Erst bei Erreichen von, beispielsweise 50% - 80%, des Volumens V1 (25) wird der' Druck wieder auf seinen Ausgangswert Pi1 (22)(eingestellte Druckdifferenz bzw. angepasste Druckdifferenz) abgesenkt. Durch dieses Manöver soll die Resistance (Strömungswiderstand von Tubus und Inspirationsschlauch) kompensiert werden. Die Rampe von beispielsweise 50 - 380 ms, bevorzugt 100 - 300 ms, besonders bevorzugt 150 - 250 ms ist dabei auch so gewählt, dass das Volumen nicht zu rasch in die Lunge gelangt, um Schäden durch Scherspannungen zu vermeiden.

Der Druckgradient kann auch nicht konstant sein, sondern einer Funktion entsprechen beispielsweise einer Sinusfunktion oder einer Exponentialfunktion oder einer Funktion die eine Anpassung an physiologische Verhältnisse ermöglicht.

Exspiratorisch wird der Druck im Schlauchsystem, wenn nötig, bis maximal auf Umgebungsdruck abgesenkt. Die Steuerung stellt sicher, dass der Druck an der Tubusspitze, den eingestellten PEEP-Druck (oder CPAP-Druck) nicht unterschreitet. Bei obstruktiven Patienten kann es sinnvoll sein, den Druck an der Tubusspitze länger oberhalb des eingestellten CPAP-Drucks gehalten. Während der Exspirations-Flussphase werden obstruktive Bereiche damit länger offengehalten.

Nach Vorgabe des niedrigen exspiratorischen Druckniveaus Pe1 (21) kann - ein höheres exspiratorisches Druckniveau Pe2 (31) vorgegeben werden.

Es kann vorgesehen werden, dass der Druckverlaut (28) und/oder der Druckverlauf (27) und/oder der Druckverlauf (29), beispielsweise in Abhängigkeit zum Fluss oder zum Volumen, vorgebbar gesteuert sind.

## Patentansprüche

1. Vorrichtung zur Beatmung eines mit einem Tubus intubierten Patienten, umfassend:
- eine Atemgasquelle (3),
- mindestens einen Sensor (15),
- eine Steuereinheit (13), wobei die Steuereinheit (13) an den mindestens einen Sensor (15) zur Erfassung eines Messwertes angeschlossen ist,
- eine Einrichtung (4, 5, 6) zur Verbindung mit dem Tubus (11), und
- einen Analysator (18), wobei die Steuereinheit (13) einen Druckgenerator (19) zur Vorgabe von wenigstens zwei von der Atemgasquelle (3) erzeugten Druckniveaus aufweist, und wobei die Vorrichtung derart ausgestaltet ist, dass ein Atemgasfluss aus der Atemgasquelle mit von dem Druckgenerator (19) vorgegebenem Druck dem Patienten zur Beatmung mittels der Einrichtung und des Tubus zugeführt wird, und
wobei der Druckgenerator (19) ausgebildet ist, folgende Verfahrensschritte auszuführen:
- in einem ersten, initialen Atemzyklus, Vorgeben des Drucks initial auf ein exspiratorisches Niveau Pe1 (21) und dann auf ein höheres inspiratorisches Niveau Pi1 (22), und
- in einem zweiten, nachfolgenden Zyklus, Anheben des Drucks von dem exspiratorischen Niveau Pe1 (21) mit einem ersten vorgebbaren Druckverlauf (24) auf ein inspiratorisches Niveau Pi2 (32), und
wobei der Druck Pi2 (32) höher ist als der Druck Pi1 (22), und
wobei die Steuereinheit (13) einen Speicher (16) für Messwerte aufweist, und
wobei der Sensor (15) zur Messung und Bestimmung eines Atemgasflusses ausgebildet ist, und
wobei der Sensor (15) mit dem Analysator (18) gekoppelt ist, und
wobei der Analysator (18) mit dem Druckgenerator (19) gekoppelt ist, und
wobei der Analysator (18) eine Integratorfunktion aufweist und dazu ausgebildet ist, durch Integration des mit dem Sensor (15) erfassten Atemgasflusses ein Volumen zu bestimmen, und
wobei der Analysator (18) ausgebildet ist, folgende Verfahrensschritte auszuführen:
- Bestimmen des sich in dem ersten Atemzyklus ergebenden Volumens V1 (25) durch Integration des mit dem Sensor (15) in dem ersten Atemzyklus erfassten Atemgasflusses, und
- Ablegen des bestimmten Volumens V1 (25) in dem Speicher (16), und
- Bestimmen des Volumens V2 (26) in dem zweiten Atemzyklus durch Integration des mit dem Sensor (15) in dem zweiten Atemzyklus erfassten Atemgasflusses, und
wobei der Druckgeneratör (19) ferner ausgebildet ist, folgende Verfahrensschritte auszuführen:
A bsenken, während des zweiten Atemzyklus, mit einem zweiten Druckverlauf (28), des Drucks von des Druck des inspiratorischen Niveaus Pi2 (32) erst dann auf den Druck des niedrigeren inspiratorischen Niveaus Pi1 (22), wenn das bestimmte Volumen V2 ' (26) einen vordefinierten Anteil des abgelegten Volumens V1 (25) erreicht hat,
so dass die weitere Inspiration im zweiten Atemzyklus unter dem Druck Pi1 (22) erfolgt.

2. Vorrichtung nach Anspruch 1 **dadurch gekennzeichnet, dass** der Druck Pi2 (32) ein vorgebbarer maximaler Druck Pmax ist.

3. Vorrichtung nach Anspruch 2 **dadurch gekennzeichnet, dass** der vorgebbare maximale Druck Pmax zwischen 1 und 6 mbar liegt.

4. Vorrichtung nach zumindest einem der vorhergehenden Ansprüche **dadurch gekennzeichnet, dass** der erste vorgebbare Druckverlauf (24) eine Druckrampe von 50 bis 380 ms ist.

5. Vorrichtung nach zumindest einem der vorhergehenden Ansprüche, **dadurch gekennzeichnet, dass** der Druckgenerator (19) ferner dazu ausgebildet ist, folgenden Verfahrensschritt auszuführen: Absenken des Drucks von dem inspiratorischen Niveau Pi2 (32) für den dem zweiten Atemzyklus nachfolgenden Atemzyklus, -wenn die Zeit, in der das im zweiten Atemzyklus bestimmte Volumen V2 (26) den vordefinierten Anteil des abgelegten Volumens V1 (25) erreicht, eine erste vorgegebene Zeit unterschreitet, wobei die erste vorgegebene Zeit eine Zeit von 200 bis 400 ms, bevorzugt von 300 ms, ist.

6. Vorrichtung nach zumindest einem der vorhergehenden Ansprüche 1 bis 4, **dadurch gekennzeichnet, dass** der Druckgenerator (19) ferner dazu ausgebildet ist, folgenden Verfahrensschritt auszuführen: Anheben, des Drucks des inspiratorischen Niveaus Pi2 (32) für den dem zweiten Atemzyklus nachfolgenden Atemzyklus, wenn die Zeit, in der das im zweiten Atemzyklus bestimmte Volumen V2 (26) den vordefinierten Anteil des abgelegten Volumens V1 (25) erreicht, eine zweite vorgegebene Zeit überschreitet, wobei die zweite vorgegebene Zeit eine Zeit von 250 bis 450 ms, bevorzugt von 350 ms, ist.

7. Vorrichtung nach zumindest einem der vorhergehenden Ansprüche **dadurch gekennzeichnet, dass** der vordefinierte Anteil 60 % bis 95 % ist.

8. Vorrichtung nach zumindest einem der vorhergehenden Ansprüche **dadurch gekennzeichnet, dass** der Druckgenerator (19) **ferner dazu ausgebildet** ist, folgende Verfahrensschritte **auszuführen:** in dem dem zweiten Atemzyklus nachfolgenden Atemzyklus,
- Absenken des Drucks von dem inspiratorischen Niveau Pi1 (22) am Ende des zweiten Atemzyklus auf das exspiratorische Niveau Pe1 (21) mit einem dritten Druckverlauf (27) und
- dann Anheben des Drucks von dem exspiratorischen Niveau Pe1 (21) auf ein höheres exspiratorisches Niveau Pe2 (31) mit einem vierten Druckverlauf (29) und
- dann Absenken des Drucks von dem höheren exspiratorischen Niveau Pe2 (31) auf das exspiratorische Niveau Pe1 (21).

9. Vorrichtung nach dem vorhergehenden Ansprüche **dadurch gekennzeichnet, dass** der Druckgenerator (19) ferner derart ausgebildet ist, dass der dritte Druckverlauf (28) und/oder der zweite Druckverlauf (27) und/oder der vierte Druckverlauf (29) vorgebbar sind.

10. Vorrichtung nach dem vorhergehenden Anspruch 8, **dadurch gekennzeichnet, dass** der Druckgenerator (19) ferner dazu ausgebildet ist, folgenden Verfahrensschritt auszuführen: Steuern des dritten Druckverlaufs (28) und/oder des zweiten Druckverlaufs (27) und/oder des vierten Druckverlaufs (29) in Abhängigkeit des Atemgasflusses oder des Volumens.

## Claims

1. A device for ventilating a patient intubated with a tube, comprising:
- a breathing gas source (3),
- at least one sensor (15),
- a control unit (13), wherein the control unit (13) is connected to the at least one sensor (15) for detecting a measurement value,
- an apparatus (4, 5, 6) for connecting to the tube (11), and
- an analyzer (18),
wherein the control unit (13) has a pressure generator (19) for specifying at least two pressure levels generated by the breathing gas source (3), and wherein the device is designed such that a breathing gas flow from the breathing gas source is fed to the patient with the pressure specified by the pressure generator (19) for ventilation by means of the apparatus and the tube, and wherein the pressure generator (19) is designed to carry out the following method steps:
- in a first, initial breathing cycle, specifying the pressure initially to an expiratory level Pe1 (21) and then to a higher inspiratory level Pi1 (22), and
- in a second, subsequent cycle, raising the pressure from the expiratory level Pe1 (21)' to an inspiratory level Pi2 (32) with a first specifiable pressure curve (24), and wherein the pressure Pi2 (32) is higher than the pressure Pi1 (22) and wherein the control unit (13) has a memory (16) for measurement values, and wherein the sensor (15) is designed to measure and determine a breathing gas flow, and wherein the sensor (15) is coupled with the analyzer (18), and wherein the analyzer (18) is coupled with the pressure generator (19), and wherein the analyzer (18) has an integrator function and is designed to determine a volume by integration of the breathing gas flow detected with the sensor (15), and the analyzer (18) is designed to carry out the following method steps:
- determining the resulting volume V1 (25) in a first breathing cycle by integration of the breathing gas flow detected with the sensor (15) in the first breathing cycle, and
- storing the determined volume V1 (25) in the memory (16), and
- determining the volume V2 (26) in the second breathing cycle by integration of the breathing gas flow detected with the sensor (15) in the second breathing gas cycle, and wherein the pressure generator (19) is further designed to carry out the following method steps:
- lowering, during the second breathing cycle, the pressure with a second pressure curve (28) from the pressure of the inspiratory level Pi2 (32) to the pressure of the lower inspiratory level Pi1 (22) only when the determined volume V2 (26) has reached a predefined proportion of the stored volume V1 (25), so that the further inspiration takes place in the second breathing cycle under the pressure Pi1 (22).

2. The device according to claim 1, **characterized in that** the pressure Pi2 (32) is a specifiable maximum pressure Pmax.

3. The device according to claim 2, **characterized in that** the specifiable maximum pressure Pmax is between 1 and 6 mbar.

4. The device according to at least one of the preceding claims, **characterized in that** the first, specifiable pressure curve (24) is a pressure ramp of 50 to 380 ms.

5. The device according to at least one of the preceding claims, **characterized in that** the pressure generator (19) is further designed to carry out the following method step: lowering the pressure from the inspiratory level Pi2 (32) for the breathing cycle following the second breathing cycle when the time in which the volume V2 (26) determined in the second breathing cycle reaches the predefined proportion of the stored volume V1 (25) falls below a first specified time, wherein the first specified time is a time of 200 to 400 ms, preferably of 300 ms.

6. The device according to at least one of the preceding claims 1. to 4, **characterized in that** the pressure generator (19) is further designed to carry out the following method step: raising the pressure of the inspiratory level Pi2 (32) for the breathing cycle following the second breathing cycle when the time in which the volume V2 (26) determined in the second breathing cycle reaches the predefined proportion of the stored volume V1 (25) exceeds a second specified time, wherein the second specified time is a time of 250 to 450 ms, preferably of 350 ms.

7. The device according to at least one of the preceding claims, **characterized in that** the predefined proportion is 60% to 95%.

8. The device according to at least one of the preceding claims, **characterized in that** the pressure generator (19) is further designed to carry out the following method steps in the breathing cycle following the second breathing cycle:
- lowering the pressure from the inspiratory level Pi1 (22) at the end of the second breathing cycle to the expiratory level Pe1 (21) with a third pressure curve (27), and
- then raising the pressure from the expiratory level Pe1 (21) to a higher expiratory level Pe2 (31) with a fourth pressure curve (29), and
- then lowering the pressure from the higher expiratory level Pe2 (31) to the expiratory level Pe1 (21).

9. The device according to the preceding claim, **characterized in that** the pressure generator (19) is further designed such that the third pressure curve (28) and/or the second pressure curve (27) and/or the fourth pressure curve (29) can be specified.

10. The device according to the preceding claim 8, **characterized in that** the pressure generator (19) is further designed to carry out the following method step: controlling the third pressure curve (28) and/or the second pressure curve (27) and/or the fourth pressure curve (29) depending on the breathing gas flow or the volume.

## Revendications

1. Dispositif pour la ventilation d'un patient intubé avec un tuyau, comportant :
- une source de gaz respiratoire (3),
- au moins un capteur (15),
- une unité de commande (13), dans lequel l'unité de commande (13) est raccordée à l'au moins un capteur (15) pour la détection d'une valeur de mesure,
- un dispositif (4, 5, 6) destiné au raccordement au tuyau (11), et
- un analyseur (18),
dans lequel l'unité de commande (13) présente un générateur de pression (19) permettant de définir au moins deux niveaux de pression produits par la source de gaz respiratoire (3), et dans lequel le dispositif est conçu de telle façon qu'un flux de gaz respiratoire est alimenté vers le patient à partir de la source de gaz respiratoire avec une pression définie par le générateur de pression (19) pour la ventilation au moyen du dispositif et du tuyau, et dans lequel le générateur de pression (19) est réalisé pour mettre en œuvre les étapes de procédé suivantes :
- dans un premier cycle respiratoire initial, définition de la pression initialement à un niveau expiratoire Pe1 (21), puis à un niveau inspiratoire plus élevé Pi1 (22), et
- dans un deuxième cycle consécutif, augmentation de la pression du niveau expiratoire Pe1 (21) avec une première courbe de pression prédéfinissable (24) à un niveau inspiratoire Pi2 (32), et dans lequel la pression Pi2 (32) est plus élevée que la pression Pi1 (22) et dans lequel l'unité de commande (13) présente une mémoire (16) pour des valeurs de mesure, et dans lequel le capteur (15) est configuré pour mesurer et déterminer un flux de gaz respiratoire, et dans lequel le capteur (15) est accouplé à l'analyseur (18), et dans lequel l'analyseur (18) est accouplé au générateur de pression (19), et dans lequel l'analyseur (18) présente une fonction d'intégrateur et est configuré pour déterminer un volume par intégration du flux de gaz respiratoire détecté à l'aide du capteur (15) et l'analyseur (18) est configuré pour mettre en œuvre les étapes de procédé suivantes :
- détermination du volume V1 (25) apparaissant dans un premier cycle respiratoire par intégration du flux de gaz respiratoire détecté dans le premier cycle respiratoire à l'aide du capteur (15) et
- enregistrement du volume V1 (25) déterminé dans la mémoire (16) et
- détermination du volume V2 (26) dans le deuxième cycle respiratoire par intégration du flux de gaz respiratoire détecté dans le deuxième cycle respiratoire à l'aide du capteur (15), et dans lequel le générateur de pression (19) est en outre configuré pour mettre en œuvre les étapes de procédé suivantes :
- réduction de la pression, pendant le deuxième cycle respiratoire avec une deuxième courbe de pression (28), de la pression du niveau inspiratoire Pi2 (32) à la pression du niveau inspiratoire moins élevé Pi1 (22), lorsque le volume V2 (26) déterminé a atteint une part prédéfinie du volume V1 (25) enregistré, de telle façon que l'inspiration suivante s'effectue sous la pression Pi1 (22) dans le deuxième cycle respiratoire.

2. Dispositif selon la revendication 1, **caractérisé en ce que** la pression Pi2 (32) est une pression maximale prédéfinissable Pmax.

3. Dispositif selon la revendication 2, **caractérisé en ce que** la pression maximale prédéfinissable Pmax est comprise entre 1 et 6 mbar.

4. Dispositif selon l'une au moins des revendications précédentes, **caractérisé en ce que** la première courbe de pression (24) prédéfinissable est une pente de pression de 50 à 380 ms.

5. Dispositif selon l'une au moins des revendications précédentes, **caractérisé, en ce que** le générateur de pression (19) est en outre configuré pour mettre en œuvre l'étape de procédé suivante : réduction de la pression à partir du niveau inspiratoire Pi2 (32) pour le cycle respiratoire consécutif au deuxième cycle respiratoire, lorsque la durée pendant laquelle le volume V2 (26) déterminé dans le deuxième cycle respiratoire atteint la part prédéfinie du volume V1 (25) enregistré est inférieure à une première durée prédéfinie, dans lequel la première durée prédéfinie est une durée de 200 à 400 ms, de préférence de 300 ms.

6. Dispositif selon l'une au moins des revendications précédentes 1 à 4, **caractérisé en ce que** le générateur de pression (19) est en outre configuré pour mettre en œuvre l'étape de procédé suivante : augmentation de la pression du niveau inspiratoire Pi2 (32) pour le cycle respiratoire consécutif au deuxième cycle respiratoire, lorsque la durée pendant laquelle le volume V2 (26) déterminé dans le deuxième cycle respiratoire atteint la part prédéfinie du volume V1 (25) enregistré est supérieur à une deuxième durée prédéfinie, dans lequel la deuxième durée prédéfinie est une durée de 250 à 450 ms, de préférence de 350 ms.

7. Dispositif selon l'une au moins des revendications précédentes, **caractérisé en ce que** la part prédéfinie est de 60% à 95%.

8. Dispositif selon l'une au moins des revendications précédentes, **caractérisé en ce que** le générateur de pression (19) est en outre configuré pour mettre en œuvre les étapes de procédé suivantes dans le cycle respiratoire consécutif au deuxième cycle respiratoire :
- réduction de la pression à partir du niveau inspiratoire Pi1 (22) à la fin du deuxième cycle respiratoire, au niveau expiratoire Pe1 (21) avec une troisième courbe de pression (27) et
- ensuite, augmentation de la pression à partir du niveau expiratoire Pe1 (21) à un niveau expiratoire Pe2 (31) plus élevé avec une quatrième courbe de pression (29) et
- ensuite, réduction de la pression à partir du niveau expiratoire Pe2 (31) plus élevé au niveau expiratoire Pe1 (21).

9. Dispositif selon la revendication précédente, **caractérisé en ce que** le générateur de pression (19) est en outre configuré de telle façon que la troisième courbe de pression (28) et/ou la deuxième courbe de pression (27) et/ou la quatrième courbe de pression (29) peuvent être prédéfinies.

10. Dispositif selon la revendication précédente 8, **caractérisé en ce que** le générateur de pression (19) est en outre configuré pour mettre en œuvre l'étape de procédé suivante : commande de la troisième courbe de pression (28) et/ou de la deuxième courbe de pression (27) et/ou de la quatrième courbe de pression (29) en fonction du flux de gaz respiratoire ou du volume.
